# EUROPÄISCHE PATENTANMELDUNG

(11) **EP 1 836 950 A1**
(43) Veröffentlichungstag der Anmeldung: **26.09.2007**
(21) Anmeldenummer: 07002881.6
(22) Anmeldetag: 10.02.2007
(51) Int. Cl.: A61B 1/07, A61B 1/227

(54) **Beleuchtungsvorrichtung für ärztliche Instrumente, insbesondere Otoskope**

(30) Priorität: 22.03.2006 DE 202006004508 U
(71) Anmelder: Kirchner & Wilhelm GmbH + Co. KG, 71679 Asperg (DE)
(72) Erfinder: Kirchner, Regina Margarete Clara, 71706 Markgröningen (DE)
(74) Vertreter: Kohler Schmid Möbus

(57) **Zusammenfassung**

Um eine Beleuchtungsvorrichtung für ärztliche Instrumente, bei welcher das Licht von einer Lichtquelle über einen Lichtleiter 3 zu einem Lichtaustritt übertragen wird, einfach herzustellen und störungssicher auszubilden, besteht der Lichtleiter 3 aus Acryl.

## Beschreibung

Die vorliegende Erfindung betrifft eine Beleuchtungsvorrichtung für ärztliche Instrumente, insbesondere Otoskope. Hierbei wird das Licht von einer Lichtquelle über einen Lichtleiter zum Lichtaustritt übertragen. Bei bekannten Otoskopen dieser Art weist das Otoskop beispielsweise einen Außentrichter auf, in welchen ein Innentrichter eingeschoben ist. Der Lichtleiter ist zwischen dem Innen- und Außentrichter angeordnet.

Herkömmlich wird ein solcher Lichtleiter aus Glasfasern hergestellt. Dabei werden sehr dünne Einzelfasern mit einem Durchmesser von 50 µm verwendet, die in Längsrichtung gebündelt werden.

Die einzelnen Fasern werden dann untereinander verklebt. Nach einem Aushärten des Klebstoffs wird der durch das Verkleben erhaltene Faserstrang auf die richtige Länge gestutzt und in die gewünschte Form gebracht, die dann in einem Ofen temperaturgehärtet wird. Danach werden die Enden des Strangs, die als Lichteintrittsfläche und als Lichtaustrittsfläche dienen, poliert und geläppt.

Es ist zu erkennen, dass die beschriebene Herstellung eines Lichtleiters sehr aufwändig ist. Während der Herstellung sind viele Einzelschritte zu durchlaufen und auch der zeitliche Aufwand für die jeweiligen Einzelschritte ist groß. Weiterhin tritt bei Glasfasern häufiger das Problem auf, dass einzelne Fasern in einem Strang beschädigt werden, die dann ein Hindernis für eine Lichtübertragung darstellen, so dass der Lichttransmissionsgrad geringer wird. Auch kann ein Aufbrechen von Verklebungen zwischen den Fasern auftreten.

Es ist eine Aufgabe der Erfindung, eine Beleuchtungsvorrichtung für ärztliche Instrumente, insbesondere Otoskope, und einen Lichtleiter für eine solche Beleuchtungsvorrichtung zur Verfügung zu stellen, wobei der Lichtleiter einfach herzustellen und störungssicher ausgebildet ist. Diese Aufgabe wird erfindungsgemäß durch die im Anspruch 1 angegebene Beleuchtungsvorrichtung.

Insbesondere besteht der Lichtleiter der Beleuchtungsvorrichtung erfindungsgemäß aus Acryl. Er wird vorzugsweise aus PMMA Granulat hergestellt. Dieses Material ist bezüglich einer erforderlichen Lichttransmission von einer Lichteintrittsfläche bis zu einer Lichtaustrittsfläche ähnlich bislang verwendeten Glasfasern.

Nach einem Festlegen der erforderlichen Form des Lichtleiters muss ein Spritzwerkzeug für einen spritztechnischen Vorgang hergestellt werden, mit welchem dann die Lichtleiter gefertigt werden. Somit ist beim Verwenden von Acryl für den Lichtleiter, wenn das Spritzwerkzeug einmal hergestellt ist, das Verfahren zum Herstellen des Lichtleiters einfacher und weniger zeit- und kostenintensiv als beim Verwenden von Glasfasern.

Weil der Lichtleiter aus Acryl kompakt aus einem Stück ausgebildet ist, ist er mechanisch stabiler als ein Lichtleiter aus einzelnen Glasfasern. Eine Beschädigung von einzelnen Fasern kann nicht erfolgen. Ebenso ist ein Aufbrechen von Verklebungen ausgeschlossen.

Die Zeichnung zeigt:
- Fig. 1: den Querschnitt eines bekannten Otoskopkopfes mit einem Lichtleiter aus Glasfasern,
- Fig. 2: die Ansicht eines Lichtleiters für eine Beleuchtungsvorrichtung gemäß der Erfindung.

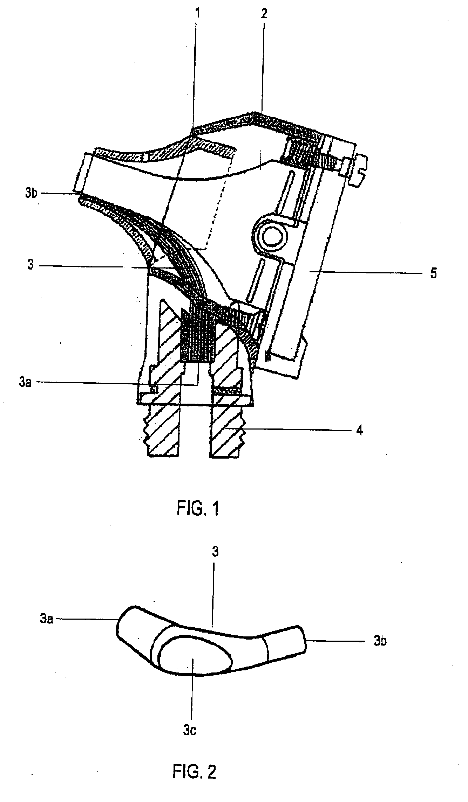

Unter Bezugnahme auf die Fig. 1 wird zuerst allgemein ein Otoskopkopf beschrieben, bei welchem ein aus Glasfasern hergestellter Lichtleiter eingebaut ist.

Der Otoskopkopf weist einen Außentrichter 1 auf, in welchen ein Innentrichter 2 eingeschoben ist. Zwischen dem Außentrichter 1 und dem Innentrichter 2 ist ein Lichtleiter 3 angeordnet. Der Lichtleiter 3 ist hier aus einzelnen Glasfasern ausgebildet gezeigt. Seine Lichteintrittsfläche 3a ist bei einem Anschlussstück 4 vorgesehen, an das eine nicht gezeigte Lichtquelle angeschlossen wird. Er verläuft zwischen dem Außentrichter 1 und dem Innentrichter 2 bis zu Enden der Trichter 1 und 2 in einer Lichtaustrittsfläche 3b. In Richtung zu ihren anderen Enden weiten sich die Trichter 1 und 2 auf. An diesen Enden ist als Optik beispielsweise eine Linse 5 vorgesehen.

Gemäß der Erfindung ist der Aufbau des Otoskopkopfes derselbe wie derjenige, der in Fig. 1 gezeigt ist, außer dass der Leichtleiter 3 aus Glasfasern durch einen Lichtleiter 3 aus Acryl ersetzt ist. Ein solcher aus Acryl bestehender Lichtleiter 3 ist in Fig. 2 gezeigt. Er ist vorzugsweise aus PMMA Granulat mit Materialeigenschaften in Bezug auf eine Lichttransmission ähnlich den Glasfasern hergestellt. Er ist als Spritzgussteil ausgebildet und hat eine Lichteintrittsfläche 3a und eine Lichtaustrittsfläche 3b. Durch die Herstellung des Lichtleiters 3 als Spritzgussteil können gewünschte Formen auf einfache Weise gefertigt werden. So kann beispielsweise durch entsprechendes Ausbilden des Spritzwerkzeugs ohne größeren Aufwand eine Ausnehmung 3c am Lichtleiter 3 ausgebildet werden, die für den Einbau des Lichtleiters 3 zwischen dem Außentrichter 1 und den Innentrichter 2 nötig sein kann. Der in Fig. 2 gezeigte Lichtleiter 3 aus Acryl ist mechanisch stabil und gegenüber Störungen weniger anfällig als ein Lichtleiter aus Glasfasern.

Die Beleuchtungsvorrichtung der vorliegenden Erfindung weist einen Lichtleiter aus Acryl auf, der einfach, schnell und kostengünstig hergestellt werden kann und zudem noch störungssicherer als ein herkömmlicher Lichtleiter aus Glasfasern ist.

## Patentansprüche

1. Beleuchtungsvorrichtung für ärztliche Instrumente, insbesondere Otoskope, wobei Licht von einer Lichtquelle über einen Lichtleiter (3) zu einem Lichtaustritt übertragen wird, **dadurch gekennzeichnet, dass** der Lichtleiter (3) aus Acryl besteht.

2. Beleuchtungsvorrichtung für ärztliche Instrumente, insbesondere Otoskope, nach Anspruch 1, **dadurch gekennzeichnet, dass** der Lichtleiter (3) aus PMMA Granulat hergestellt ist.

3. Beleuchtungsvorrichtung für ärztliche Instrumente, insbesondere Otoskope, nach Anspruch 1 oder 2, **dadurch gekennzeichnet, dass** der Lichtleiter (3) als Spritzgussteil ausgebildet ist.
